# EUROPEAN PATENT APPLICATION

(11) **EP 4 606 315 A1**
(43) Date of publication of application: **27.08.2025**
(21) Application number: 23880167.4
(22) Date of filing: 17.10.2023
(51) Int. Cl.: A61B 8/00, A61B 8/12, A61B 5/00

(54) **SCANNING TIP FOR PHOTOACOUSTIC-ULTRASOUND MINI PROBE**

(30) Priority: 17.10.2022 KR 20220133574; 13.10.2023 KR 20230136980
(71) Applicant: Ulsan National Institute of Science and Technology (UNIST), Eonyang-eup Ulju-gun Ulsan 44919 (KR)
(72) Inventor: YANG, Joon-Mo, Ulju-gun, Ulsan 44919 (KR)
(74) Representative: Studio Torta S.p.A.
(86) International application number: PCT/KR2023/016017
(87) International publication number: WO 2024/085590

(57) **Abstract**

According to the present disclosure, an original probe design was derived which enables an arrangement structure of optical and ultrasonic elements and the acquisition of optical-resolution photoacoustic endoscopic images in a situation where one or two transducers having acoustic focusing ability are arranged in a symmetrical structure with respect to a laser beam axis so as to have a synthetic acoustic focusing ability, wherein the arrangement structure of optical and ultrasonic elements allows an ultrasonic beam to be emitted collinear with a laser beam axis along which a laser beam is emitted, within a limited probe space required in the relevant field, by combining only one each of an optical fiber, a GRIN lens, and a prism commonly used in the field.

## Description

### Technical Field

The present disclosure relates to a scanning tip for a photoacoustic-ultrasound mini-probe that may be implemented having a thin long probe shape like an ultrasonic endoscope currently used in clinical practice and may be used for a medical tomography endoscope device capable of being inserted into a subject to provide a tomographic image of an area inside the subject.

### Background Art

The present disclosure relates to a so-called integrated photoacoustic and ultrasonic endoscopy (PAE-EUS) mini-probe technology that may simultaneously provide photoacoustic imaging information while maintaining a function of general endoscopic ultrasound.

### Disclosure of Invention

### Technical Problem

Conventional photoacoustic-ultrasonic probes have a problem in that a photoacoustic axis does not overlap an ultrasonic axis or an overlapping range is very narrow.

### Solution to Problem

According to the present disclosure, an original probe design which enables an arrangement structure of optical and ultrasonic elements and the acquisition of optical-resolution photoacoustic endoscopic images by the way that ultrasonic beams may be emitted overlappingly (that is, collinearly) with respect to a laser beam axis along which the laser beams are emitted, in a situation where one or two transducers with acoustic focusing capability are arranged symmetrically with respect to the laser beam axis, to be described later below, to have a synthetic acoustic focusing capability, within the limited probe space described above, by combining only an optical fiber, a GRIN lens, and a prism, has been derived.

### Advantageous Effects of Invention

According to the unique structure and manufacturing method proposed by the present disclosure as described above, it is possible to simultaneously obtain optical-resolution photoacoustic images and traditional ultrasonic images in a space of 0.7 to 1.5 mm in diameter and 10 mm or less in length of a rigid distal scanning tip, while an optical axis is perfectly collinear with an acoustic axis, without any damage to optical elements that may be caused by a pulsed laser beam passing through a relevant region. In addition, as an ultrasonic transducer is placed on a surface of a probe, the ultrasonic transducer comes into more direct contact with a surrounding acoustic matching medium, and accordingly, a probability that bubbles and so on adhere to the surface of the ultrasonic transducer during actual use, which may interfere with the related imaging procedure, is significantly reduced.

The unique probe structure proposed by the present disclosure is mainly targeted at endoscopes for diagnosing digestive diseases but may be applied to cardiovascular disease diagnosis applications that require much higher probe miniaturization, as well as various other endoscope fields.

### Brief Description of Drawings

FIG. 1A is a schematic view illustrating a distal structure of a photoacoustic-ultrasonic mini-probe according to an embodiment.
FIG. 1B is a cross-sectional view of the probe of FIG. 1A taken along an x-z plane which includes a central axis of the probe.
FIG. 1C is a cross-sectional view taken along an x-y plane at a point A-A' of the probe illustrated in FIG. 1B.
FIG. 2A to FIG. 2N are views illustrating a process of manufacturing a distal structure of the photoacoustic-ultrasonic mini-probe presented in FIG. 1, according to an embodiment.
FIG. 3A is a view illustrating only a transducer base and two ultrasonic transducers among the components illustrated in FIG. 1B.
FIG. 3B is a side view of an ultrasonic transducer implemented to have an acoustic focusing capability, according to an embodiment.
FIG. 3C is a plan view of FIG. 3B taken from above.
FIG. 4 is a view illustrating a state of a photoacoustic-ultrasonic mini-probe implemented from a scanning tip to a base portion of a probe, according to an embodiment.
FIG. 5 is a schematic view illustrating a method of arranging wires of an ultrasonic transducer, according to an embodiment.

### Best Mode for Carrying out the Invention

The present disclosure may be modified in various ways and has various embodiments, and certain embodiments are illustrated in the drawings and described in detail. Effects and features of the present disclosure and a method for achieving the effects and features will become clear with reference to the embodiments described in detail below together with the drawings. However, the present disclosure is not limited to the embodiments disclosed below but may be implemented in various forms.

Hereinafter, embodiments of the present disclosure are described in detail with reference to the attached drawings, and when description is made with reference to the drawings, the same or corresponding components are assigned the same reference numerals and redundant descriptions thereof are omitted.

In the following embodiments, the terms first, second, and so on are not used in a limited sense but are used for the purpose of distinguishing one component from another component.

In the following embodiments, the singular expression includes the plural expression unless the context clearly indicates otherwise.

In the following embodiments, the terms "include" or "have" mean that a feature or component described in the specification exists, and do not preclude the possibility that one or more other features or components may be added.

In the embodiments below, when a component is said to be "connected" to another component, this includes not only being directly connected to the other component, but also being indirectly connected to the other component through another component.

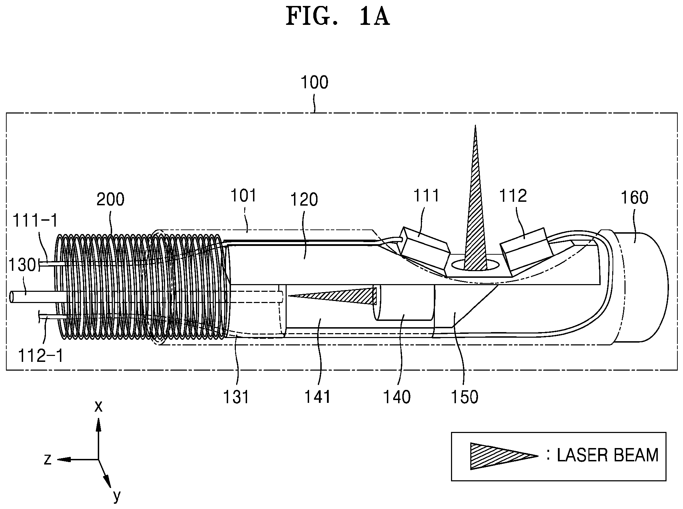

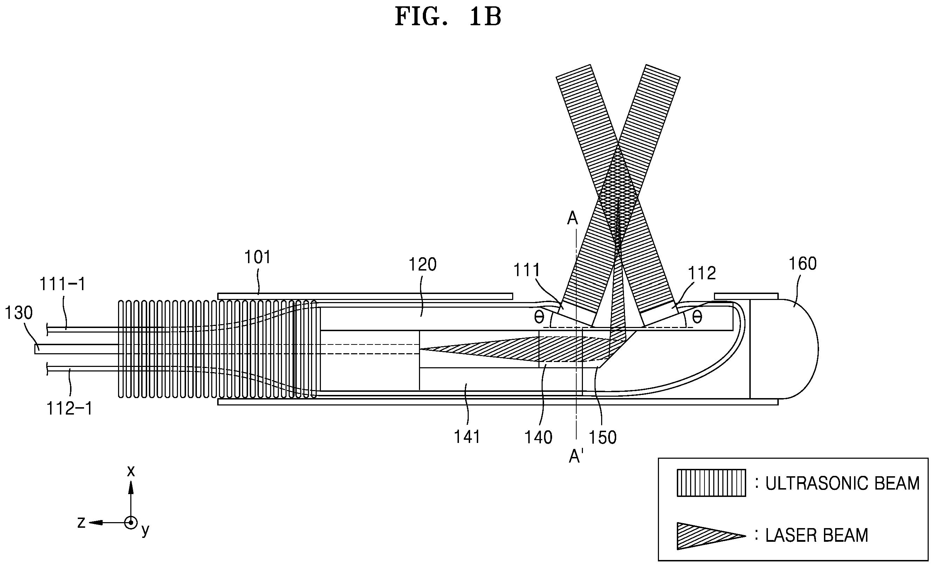

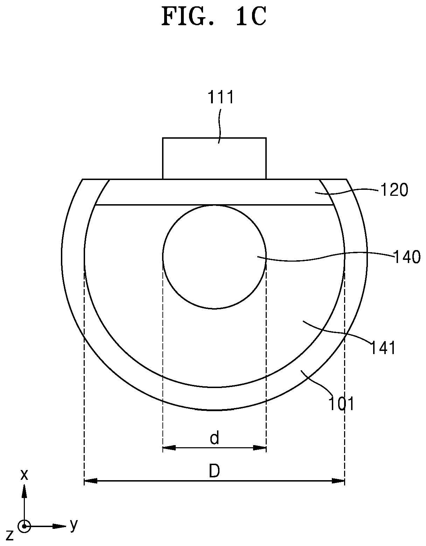

FIG. 1A is a schematic view illustrating a distal structure of a photoacoustic-ultrasonic mini-probe having both an optical-resolution photoacoustic imaging function and an ultrasonic imaging function while an optical axis is collinear with an acoustic axis, according to an embodiment, that is, a schematic view illustrating the entire configuration of a scanning tip 100 and the related operating concept. FIG. 1B is a cross-sectional view of the probe of FIG. 1A taken along an x-z plane which includes a central axis of the probe, and FIG. 1C is a cross-sectional view of the probe of FIG. 1B taken along an x-y plane at point A-A' of the probe illustrated in FIG. 1B.

Referring to FIGS. 1A to 1C, the scanning tip 100 of the optical-resolution photoacoustic-ultrasonic mini-probe derived by the present disclosure includes a transducer base 120, a first ultrasonic transducer 111, a second ultrasonic transducer 112, an optical fiber 130, a GRIN (gradient-index) lens 140, and a prism 150. The first ultrasonic transducer 111 and the second ultrasonic transducer 112 are respectively arranged on two inclined surfaces formed to have a preset inclination angle θ with respect to an axis z of an endoscope probe. In this case, the first ultrasonic transducer 111 and the second ultrasonic transducer 112 may be arranged symmetrically with respect to a central axis of a through-hole of the transducer base 120 from which a laser beam is emitted. The optical fiber 130 may receive a laser pulse, which is emitted from a laser light source (not illustrated) provided separately in the outside, from a device to which the probe is connected, and guide the laser pulse to the scanning tip 100. The position of the optical fiber 130 may be fixed by the optical fiber housing 131. An end of the optical fiber 130 may be placed at a rear space of the first ultrasonic transducer 111 and the second ultrasonic transducer 112 with respect to the transducer base 120. The GRIN lens 140 enables the laser beam emitted from the end of the optical fiber 130 to be focused on a preset working distance. The GRIN lens 140 may be fixed in position by the GRIN lens housing 141. The prism 150 may cause the laser beam, which is refracted in the form of being collected by the GRIN lens 140, to change its direction by 90° with respect to a central axis of the probe.

The components described above are surrounded by a scanning tip casing 101, and a torque coil 200 having a preset length is connected to an end of the scanning tip casing 101, and a first micro-coaxial cable 111-1 connected to the first ultrasonic transducer 111, a second micro-coaxial cable 112-1 connected to the second ultrasonic transducer 112, and the optical fiber 130 pass through the inside of the torque coil 200. In addition, an epoxy portion 160 is formed at the other end of the scanning tip casing 101 to prevent an acoustic matching medium composed of a fluid, such as water or oil, from penetrating into the inside of the scanning tip 100.

The optical fiber 130 located inside the torque coil 200 that is formed to range up to a proximal portion of the probe may guide the laser pulse received from a device connected to the probe to the scanning tip 100. When reaching the distal end of the optical fiber 130, the laser pulse is emitted at an angle determined according to an optical numerical aperture (NA) of the optical fiber 130. Thereafter, the laser pulse travels in an inner space of the GRIN lens housing 141 filled with air to an incident surface of the GRIN lens 140, is refracted by a lens effect within the GRIN lens 140, and finally travels inside the prism 150, changes its direction by 90° by a total reflection principle, and is focused into an acoustic matching medium in which the probe is immersed. That is, because the laser beam is reflected from an inclined surface of the prism 150 by the total reflection principle, a space forming a boundary with the inclined surface of the prism 150 has to be filled with air. That is, an epoxy portion 160 serves to trap the air inside the scanning tip 100. However, when reflective coating is formed on the inclined surface of the prism 150, the total reflection principle does not need to be applied, and accordingly, it does not necessarily have to come into contact with air. For reference, the prism 150 illustrated in FIG. 1A and FIG. 1B has a part of one leg removed to provide a path for the second micro-coaxial cable 112-1 connected to the second ultrasonic transducer 112 to pass therethrough, but the present disclosure is not limited thereto.

When the scanning tip 100 illustrated in FIG. 1A and FIG. 1B is used, an optical focusing capability is provided to enable optical-resolution photoacoustic imaging to be performed, and because the first ultrasonic transducer 111 is symmetric to the second ultrasonic transducer 112, a certain level of acoustic focusing capability also can be achieved during an ultrasonic imaging process. Also, in order to maximize a signal-to-noise ratio in an optical-resolution photoacoustic imaging mode, it is preferable to form a focus of the laser beam at a point where the ultrasonic beam emitted by the first ultrasonic transducer 111 and the ultrasonic beam emitted by the second ultrasonic transducer 112 intersect each other. In this case, the working distance of an endoscopic probe can be adjusted by an inclination angle 8, by which the first ultrasonic transducer 111 and the second ultrasonic transducer 112 are attached, a pitch of the GRIN lens 140, and a distance between a tip of the optical fiber 130 and the GRIN lens 140, and may be optimized through appropriate adjustment thereof according to each application. Also, when such a high-resolution photoacoustic image at an optical-resolution level is not necessary, the GRIN lens 140 may be excluded.

### Mode for the Invention

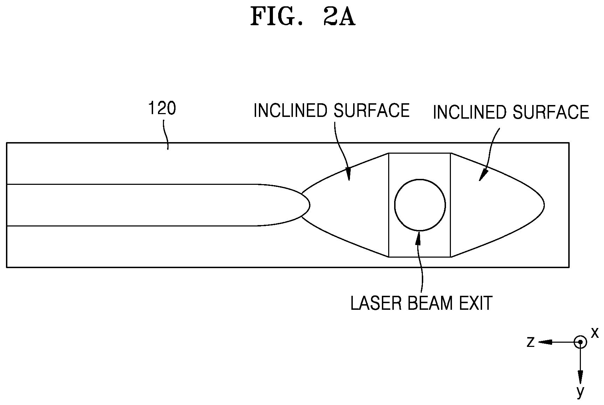

Hereinafter, a method of actually assembling and manufacturing the scanning tip 100 is described in 11 steps with reference to FIG. 2A to FIG. 2N.

As illustrated in FIG. 2A, which is a plan view, the transducer base 120 machined into a shape to have inclined surfaces symmetrical to each other with respect to an exit (that is, a through-hole) from which a laser beam is emitted according to the shape illustrated in FIG. 1A is prepared in step 1. Here, a diameter of the exit from which the laser beam is emitted has to be equal to or less than a width of the prism described below.

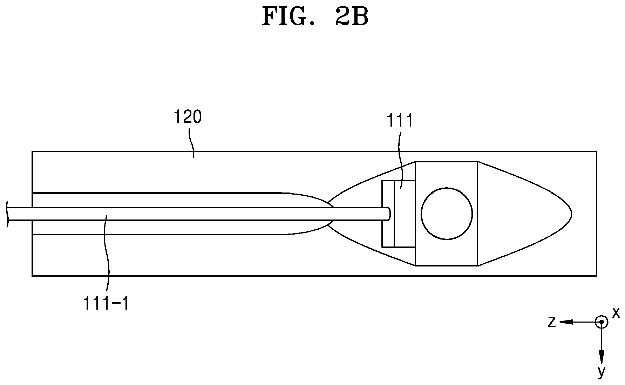

In step 2 illustrated in FIG. 2B which is a plan view, an adhesive is applied to one inclined surface to which the first ultrasonic transducer 111 and the first micro-coaxial cable 111-1 connected to each other is attached.

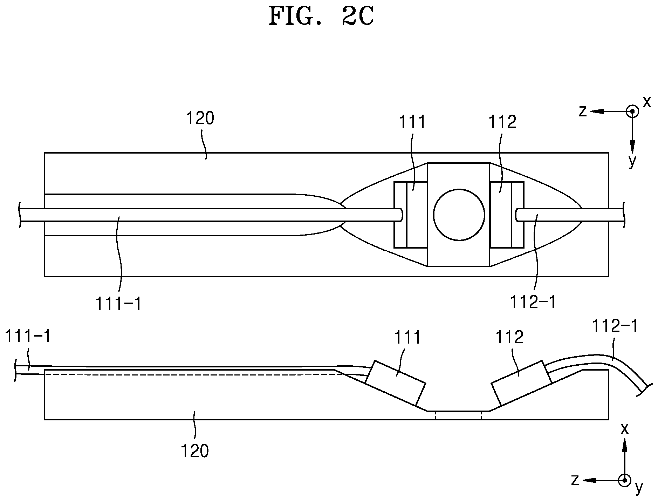

An assembly process is described in detail with reference to FIG. 2C to FIG. 2H which illustrate views taken from two different directions.

In step 3 illustrated in FIG. 2C illustrating a plan view and a side view, an adhesive is applied to an opposite inclined surface, which is opposite to an inclined surface to which the first ultrasonic transducer 111 is attached, and the second ultrasonic transducer 112 and the second micro-coaxial cable 112-1 connected to each other are attached to the opposite inclined surface.

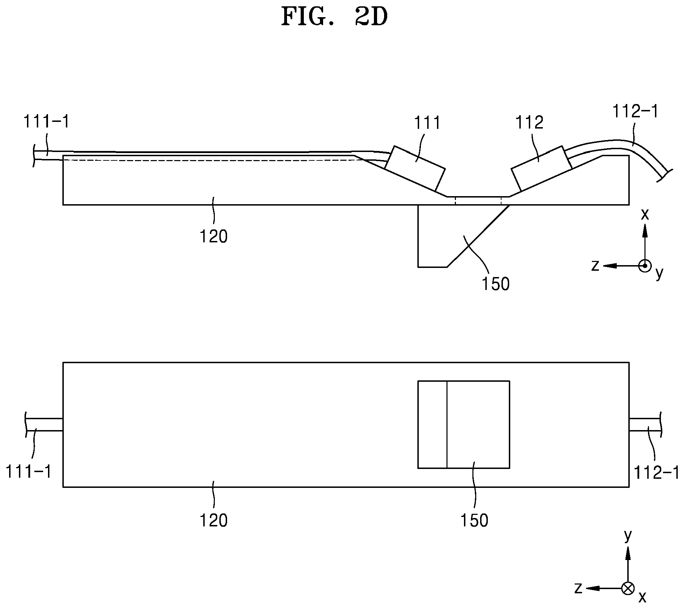

In step 4 illustrated in FIG. 2D illustrating a side view and a bottom view, an adhesive is applied to an opposite surface (a rear surface) of the transducer base 120 to which the first ultrasonic transducer 111 and the second ultrasonic transducer 112 are attached, and the prism 150 is attached the opposite surface. In this case, a position of the prism 150 has to be accurately determined in such a way that one of two vertical surfaces of the prism 150 includes a laser beam exit, and in addition, an edge of the laser beam exit (that is, a through-hole) has to be sealed with an adhesive so that an acoustic matching medium does not permeate into an optical system inside the scanning tip 100.

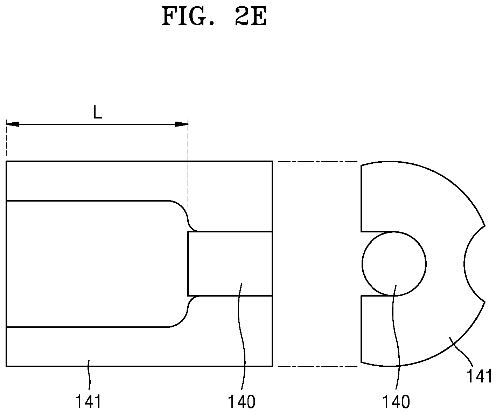

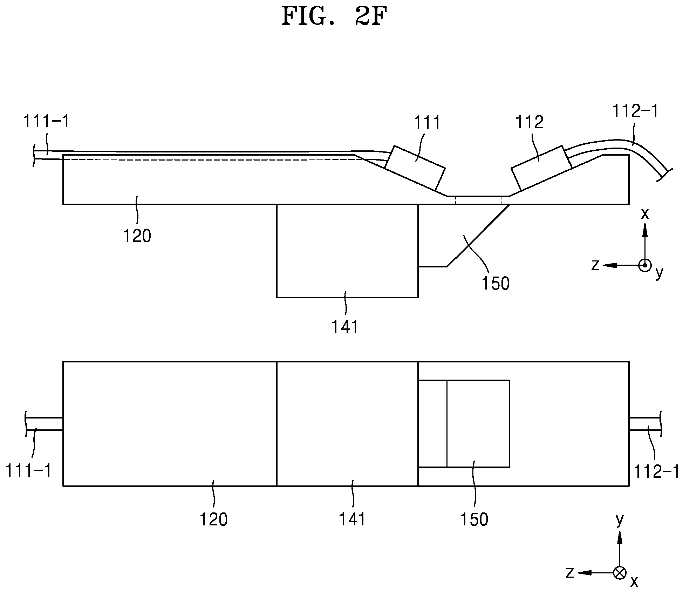

Meanwhile, as illustrated in FIG. 2E illustrating a plan view and a side view, a GRIN lens module composed of the GRIN lens 140 and the GRIN lens housing 141 pre-prepared in a separate step is placed in close contact with the other surface of the two vertical surfaces of the prism 150 according to step 5 illustrated in FIG. 2F, which is a side view and a bottom view. In this case, it is preferable to apply an adhesive to a flat surface on which the GRIN lens housing 141 is coupled to the transducer base 120 rather than to a surface on which the GRIN lens 140 is in contact with the prism 150.

As illustrated in the plan view on the left side of FIG. 2E, a length of the GRIN lens housing 141 of the GRIN lens module may be formed to be much longer than a length of the GRIN lens 140 by a length L, which is to set a desired working distance by appropriately controlling the length L. A parameter D (see FIG. 1C) of the GRIN lens module illustrated in the right side view on the right side of FIG. 2E represents a diameter of the GRIN lens module, and a parameter d (see FIG. 1C) represents a diameter of the GRIN lens 140. As illustrated in FIG. 2E, the GRIN lens housing 141 may partially surround the GRIN lens 140 without completely surrounding the GRIN lens 140. This may be understood as one side of the GRIN lens module is removed by a thickness of (D-d)/2. By doing in this way, a portion in which the GRIN lens module is partially removed may be disposed onto the transducer base 120. Through this design, the position of the GRIN lens can be precisely placed on a central axis of the scanning tip 100.

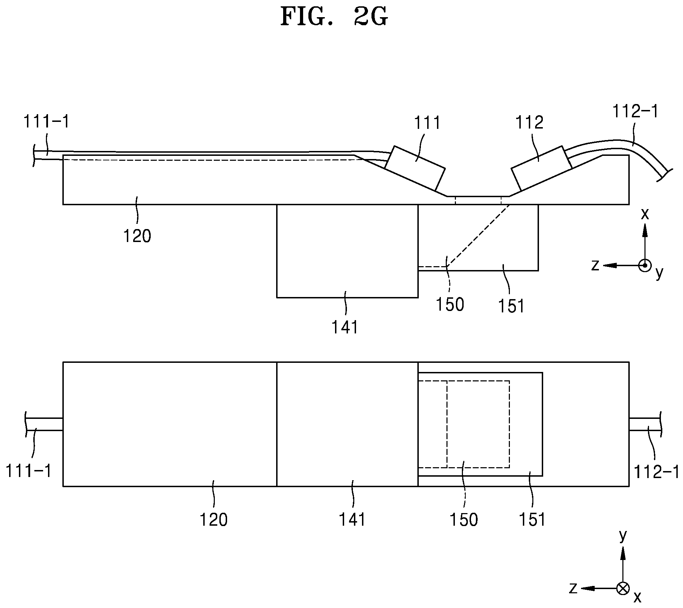

In step 6 illustrated in FIG. 2G illustrating a side view and a bottom view, the prism 150 is covered by a prism cover 151 having a cross-section in the shape of a Korean letter " 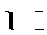 " to prevent foreign substances from adhering to an inclined surface of the prism 150 during a subsequent assembly process. The step 6 is not essential, and accordingly, the step 6 may be omitted.

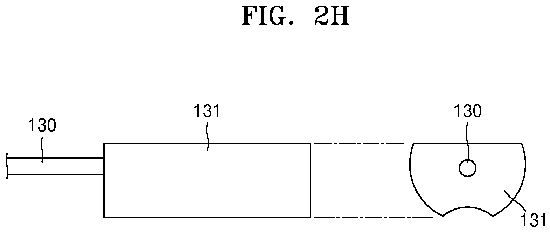

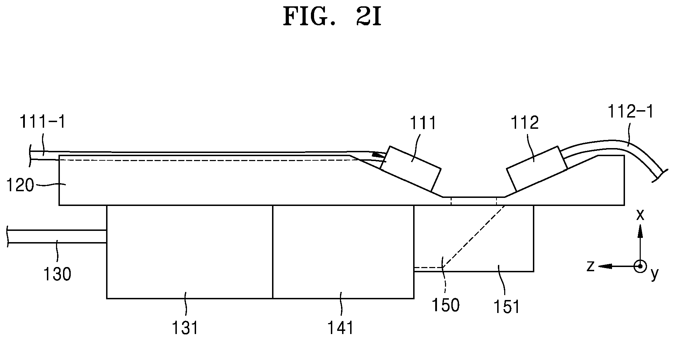

Meanwhile, as illustrated in FIG. 2H illustrating a side view and a front view, an optical fiber module composed of an optical fiber 130 and the optical fiber housing 131 pre-prepared in a separate step is attached to the remaining flat surface of the transducer base 120 so as to be in close contact with the GRIN lens module in step 7 illustrated in FIG. 2I illustrating a side view. The optical fiber housing 131 of the optical fiber module may be partially removed by a thickness of (D-d)/2, as in the case of the GRIN lens module described above. This is to ensure that an end of the optical fiber 130 is aligned with a central axis of the GRIN lens 140. In a situation where a value of the length L described above is determined in advance, the end of the optical fiber 130 has to be placed exactly on an end surface of the optical fiber housing 131 (that is, a surface indicated in the front view of FIG. 2H).

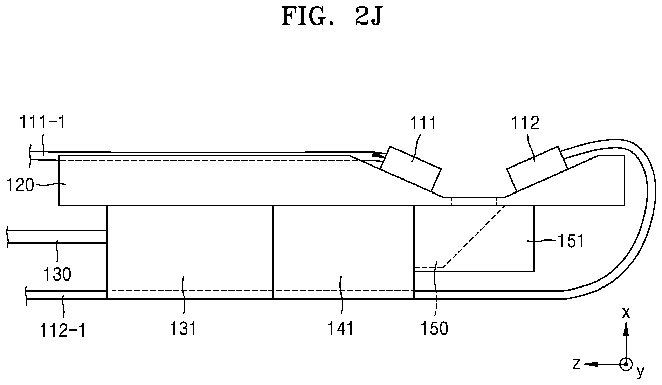

In step 8 illustrated in FIG. 2J which is a side view, the second micro-coaxial cable 112-1 is bent to pass to face away from the first ultrasonic transducer 111 and the second ultrasonic transducer 112 with respect to the transducer base 120, and is arranged and fixed along grooves pre-formed (or pre-defined) along one surface of the GRIN lens housing 141 and one surface of the optical fiber housing 131. As explained, the grooves may be pre-formed (or pre-defined) on the surfaces of the GRIN lens housing 141 and the optical fiber housing 131 such that a wire may pass through the grooves to be buried therein, but the present disclosure is not limited thereto, and the grooves may not be formed.

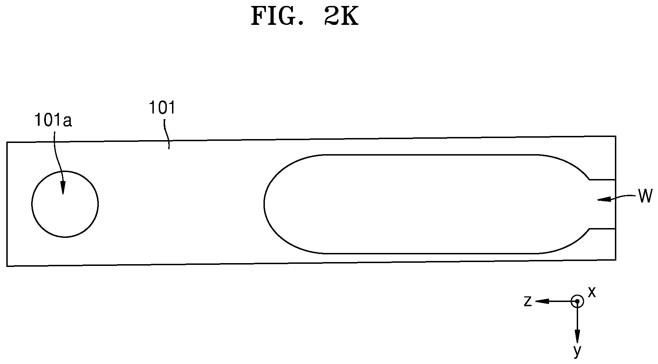

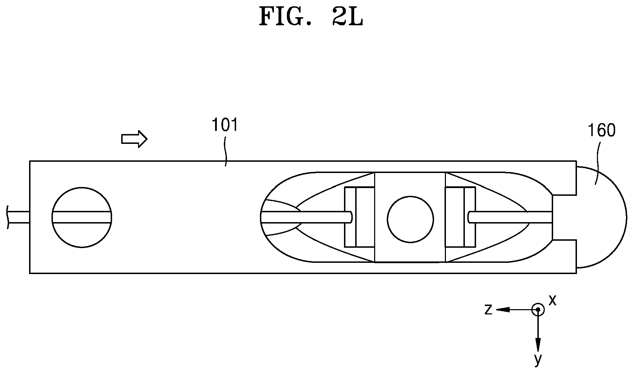

Thereafter, as illustrated in a plan view of FIG. 2K, the scanning tip casing 101 of a tube shape having a preset length and an open region on a part of a side surface is prepared in advance in a separate step, and in step 9 illustrated in FIG. 2L, the components assembled in the previous step are moved in the direction of an arrow and the components assembled in the previous step are inserted into the scanning tip casing 101 and fixed by an adhesive. The scanning tip casing 101 has an open region on a side surface, and a through-hole of the transducer base 120, the first ultrasonic transducer 111, and the second ultrasonic transducer 112 are arranged to correspond to the open region. When inserting the components assembled in the previous step into the scanning tip casing 101, the optical fiber 130 having a preset length, the first micro-coaxial cable 111-1, and the second micro-coaxial cable 112-1 have to be passed into the scanning tip casing 101.

The scanning tip casing 101 may have an open portion W formed in an open shape at the end, and the opening portion W is formed in advance to allow the second ultrasonic transducer 112 to pass therethrough smoothly when the components assembled in the previous step are inserted into the opening portion W. Also, in some cases, for example, when an inner diameter of the scanning tip casing 101 is sufficiently large, the shaping process of the relevant opening portion W may be omitted.

When the components assembled in the previous step are inserted into the scanning tip casing 101 and fixed by an adhesive, the adhesive serves not only to fix the components but also to prevent an acoustic matching medium in which a probe will be immersed in the future from flowing into the probe, and accordingly, the adhesive is filled in all gaps to seal all the gaps firmly. The epoxy portion 160 performs the sealing at an end point of the probe. The epoxy portion 160 may seal not only the end of the scanning tip casing 101 but also the entire opening portion W. For reference, the scanning tip casing 101 could have an epoxy injection hole 101a, which will be explained later.

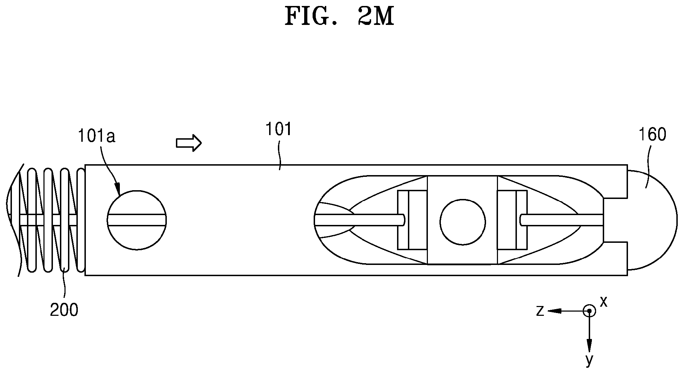

In step 10 illustrated in a plan view of FIG. 2M, the torque coil 200 is inserted into the scanning tip casing 101 and fitted into the scanning tip casing 101. Of course, an appropriate amount of epoxy may be applied to an end of the torque coil 200 for adhesion to the scanning tip casing 101 just before the fitting process.

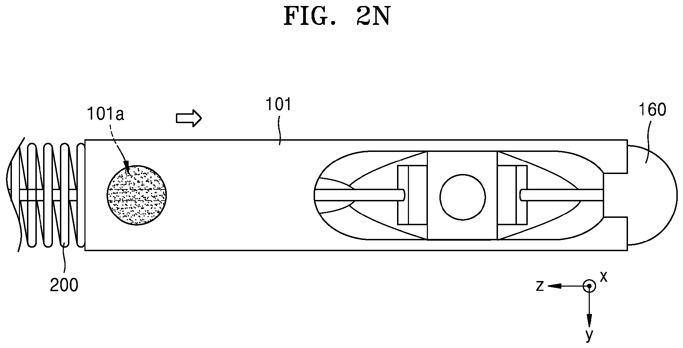

In step 11 illustrated in a plan view of FIG. 2N, by additionally applying epoxy through the epoxy injection hole 101a, which is pre-formed near the other end of the scanning tip casing 101, the epoxy can be injected into at least part of a space in the scanning tip casing (101), and accordingly, an acoustic matching medium, in which the probe will be immersed in the future, is prevented from flowing into an inner space of the scanning tip 100 through an internal channel of the torque coil 200. Also, the epoxy injection hole may be omitted.

The method of manufacturing a distal structure of a photoacoustic-ultrasonic mini-probe according to an embodiment of the present disclosure is described above. Because the method described above is only one embodiment, the described order may be partially changed, and in some cases, some processes may be omitted or other processes may be added.

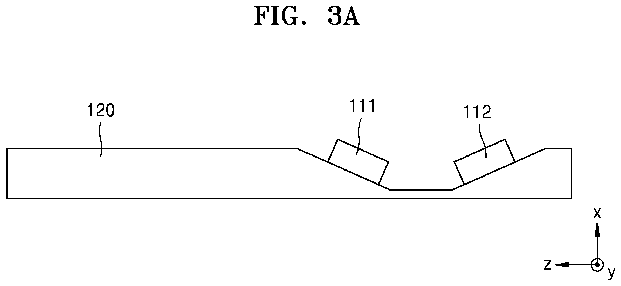

As described above, although the scanning tip with a synthetic acoustic focusing capability in a direction that is collinear with an axis from which a laser beam is emitted by using two ultrasonic transducers is presented according to one embodiment, the scanning tip of the photoacoustic-ultrasonic mini-probe according to the present disclosure may also be implemented based on a single focused ultrasonic transducer. This is described below with reference to FIG. 3A to FIG. 3C.

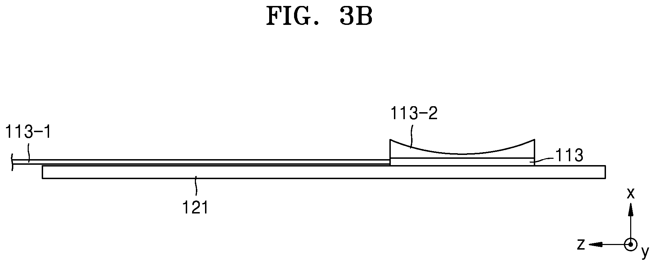

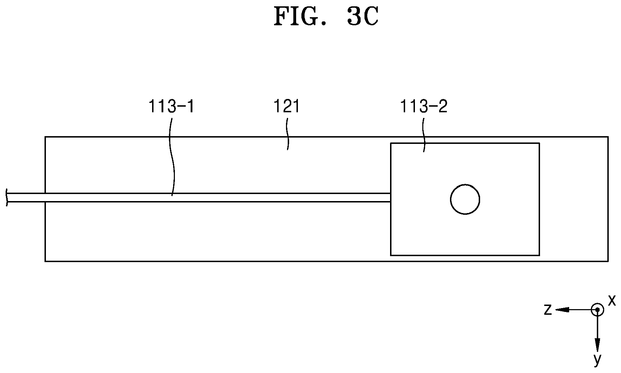

First, FIG. 3A is a view illustrating only the transducer base 120, the first ultrasonic transducer 111, and the second ultrasonic transducer 112 among the components illustrated in FIG. 1B, and FIG. 3B and FIG. 3C are views schematically illustrating a case where an ultrasonic transducer having a single piezoelectric element with an acoustic focusing capability is provided, unlike FIG. 3A. It may be understood that FIG. 3B is a side view and FIG. 3C is a plan view. As illustrated in FIG. 3B and FIG. 3C, a planar piezoelectric element 113 is provided on a planar transducer base 121 having a flat shape, and an acoustic lens 113-2 is bonded thereon to implement a scanning tip with an acoustic focusing capability. In this case, an electric signal input to and output from the planar piezoelectric element 113 is transmitted through a planar piezoelectric element cable 113-1, and a hole located at the center of the acoustic lens 113-2 illustrated in FIG. 3C is an exit through which a laser beam is emitted. An embodiment based on the single piezoelectric element may also be combined with other optical elements as illustrated in FIG. 1.

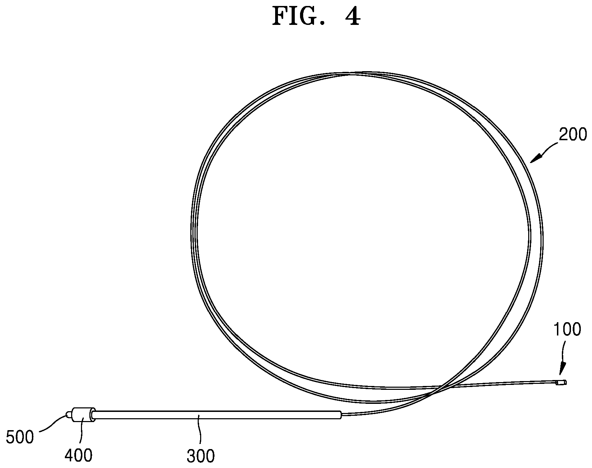

FIG. 4 is a view illustrating a state of a photoacoustic-ultrasonic mini-probe implemented from a scanning tip to a proximal portion of the probe, according to an embodiment.

The present disclosure is primarily intended to present a structure of a scanning tip capable of being used for a photoacoustic-ultrasonic mini-probe that may solve the problem described above, but various embodiments of the scanning tip presented above may additionally include several additional components illustrated in FIG. 4. FIG. 4 illustrates a torque coil 200 described above terminated in a form that includes a shaft 300 having a preset length to suit an application, an FC/PC connector 400, and a ceramic ferrule 500. In some cases, a length of the shaft 300 may determine a scan length of a three-dimensional pullback scan, and in another case, a ball bearing module may be added therearound. In addition, in another embodiment, the FC/PC connector 400 may be omitted and only the ceramic ferrule 500 may be included. In an embodiment that includes both the FC/PC connector 400 and the ceramic ferrule 500, an electrical path may also be added simultaneously to both components through a method such as conductive plating.

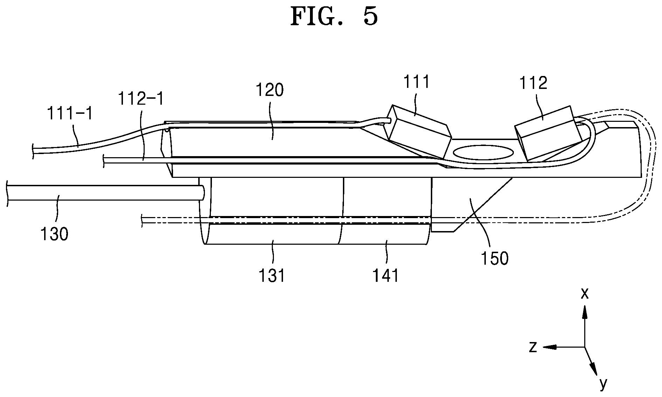

FIG. 5 is a schematic diagram illustrating a method of arranging wires of an ultrasonic transducer differently, according to an embodiment.

In FIG. 1A, FIG. 1B, FIG. 2J, FIG. 2L, FIG. 2M and FIG. 2N, the second micro-coaxial cable 112-1 connected to the second ultrasonic transducer 112 is illustrated as being arranged to pass along an outer center of the GRIN lens housing 141 and the optical fiber housing 131. However, when there is sufficient space, the second micro-coaxial cable 112-1 connected to the second ultrasonic transducer 112 may also pass through one side of the transducer base 120 as illustrated in FIG. 5. In addition, the second micro-coaxial cable 112-1 connected to the second ultrasonic transducer 112 may also pass along a path illustrated by two point-chain lines in FIG. 5, that is, through one side (+y direction) of the GRIN lens housing 141 and the optical fiber housing 131.

Because of dimensional restrictions (diameter: 0.7-1.5 mm, rigid distal length: less than 10 mm) that are commonly required in the relevant application fields of photoacoustic-ultrasonic mini-probes or catheters, it is important to effectively arrange optical and ultrasonic elements, which have to be provided, inside a distal end thereof, so-called the scanning tip. When a laser beam (that is, an optical axis) emitted from the distal end toward a lateral direction is misaligned with an acoustic axis formed by an ultrasound beam, and as a result, when tissues to be examined are not located at a distance where a laser beam axis intersects the acoustic axis, the detected signal is significantly reduced.

Also, when the related sound waves are transmitted via one or more reflective surfaces in such a configuration where an ultrasonic transducer is placed deep inside a probe rather than on a probe surface, and, serious distortion occurs in the sound waves during the sound waves propagate through a narrow space, and problems, such as bubbles forming in a space inside the probe, may easily occur because the ultrasonic transducer is placed too deep inside the probe. In addition, it is not easy to prevent related optical components from being damaged despite the pulse laser with high instantaneous peak power in a limited space and to have an optical-resolution photoacoustic imaging capability that requires high-level optical focusing.

Of course, it would be also conceptually possible to reduce the size of any endoscopic probe with any type of structure, but in reality, it is never easy to actually implement the endoscopic probe within a limited dimension. For example, even when only the problem (that is, a process of arrangement) of designating a path for a wire connected to an ultrasonic transducer is considered, the related task may seem easy in concept, but the actual implementation is never simple. Because the ultrasonic transducer is approximately 1 mm or less in diameter and 0.3 mm or less in thickness, in a state where the micro-coaxial cable attached to the transducer for inputting and outputting electrical signals is approximately 150 µm or more in thickness, while the dimension of the entire endoscope probe is 0.7-1.5 mm in diameter, it is never a trivial matter to allocate a space for a cable having a thickness of 150 µm.

However, according to the unique structure and manufacturing method proposed by the present disclosure as described above, it is possible to simultaneously realize optical-resolution photoacoustic images and traditional ultrasonic images in a space of 0.7 to 1.5 mm in diameter and 10 mm or less in length of the distal scanning tip, while an optical axis is perfectly collinear with an acoustic axis, without any damage to the optical elements caused by the pulsed laser beam passing through a relevant region. In addition, because the ultrasonic transducer is placed on a surface of the probe, the ultrasonic transducer comes into more direct contact with the surrounding acoustic matching medium, and accordingly, the probability that bubbles and so on adhere to the surface of the ultrasonic transducer during actual use to interfere with the related imaging procedure is significantly reduced.

### Industrial Applicability

The unique probe structure proposed by the present disclosure is mainly suggested to target endoscopy for diagnosing digestive diseases but may be applied to cardiovascular disease diagnosis applications that require much higher probe miniaturization, as well as other various endoscopic fields.

## Claims

1. A scanning tip for a photoacoustic-ultrasonic mini-probe, the scanning tip comprising:
a transducer base having a through-hole and having a first inclined surface located on one side of the through-hole and a second inclined surface located on the other side of the through hole to be mutually symmetrical about a central axis of the through-hole;
a first ultrasonic transducer arranged on the first inclined surface;
a second ultrasonic transducer arranged on the second inclined surface;
an optical fiber having an end arranged to face away from the first ultrasonic transducer and the second ultrasonic transducer with respect to the transducer base; and
a prism configured to reflect a laser beam emitted from the end of the optical fiber to pass through the through-hole.

2. The scanning tip for the photoacoustic-ultrasonic mini-probe of claim 1, further comprising a GRIN lens interposed between the end of the optical fiber and the prism.

3. The scanning tip for the photoacoustic-ultrasonic mini-probe of claim 1, further comprising:
a first micro-coaxial cable connected to the first ultrasonic transducer; and
a second micro-coaxial cable connected to the second ultrasonic transducer;
wherein the first micro-coaxial cable is arranged in a direction to the first ultrasonic transducer and the second ultrasonic transducer with respect to the transducer base, and the second micro-coaxial cable is bent to pass to face away from the first ultrasonic transducer and the second ultrasonic transducer with respect to the transducer base.

4. The scanning tip for the photoacoustic-ultrasonic mini-probe of claim 3, further comprising:
a GRIN lens interposed between the end of the optical fiber and the prism; and
a GRIN lens housing configured to fix the GRIN lens;
wherein the second micro-coaxial cable is arranged in a groove defined in the GRIN lens housing.

5. The scanning tip for the photoacoustic-ultrasonic mini-probe of claim 3, further comprising:
an optical fiber housing configured to fix the optical fiber,
wherein the second micro-coaxial cable is arranged in a groove defined in the optical fiber housing.

6. The scanning tip for the photoacoustic-ultrasonic mini-probe of claim 1, further comprising a scanning tip casing configured to accommodate the transducer base, the first ultrasonic transducer, the second ultrasonic transducer, the optical fiber, and the prism inside the scanning tip casing, and has an opening region corresponding to the through-hole, the first ultrasonic transducer, and the second ultrasonic transducer.

7. The scanning tip for the photoacoustic-ultrasonic mini-probe of claim 6, wherein the scanning tip casing has a tube shape of a preset length, and has, on a side, the opening region corresponding to the through-hole, the first ultrasonic transducer, and the second ultrasonic transducer.

8. The scanning tip for the photoacoustic-ultrasonic mini-probe of claim 7, wherein the scanning tip casing has an opening portion of which an end is open.

9. The scanning tip for the photoacoustic-ultrasonic mini-probe of claim 8, further comprising an epoxy portion configured to seal the end of the scanning tip casing and the opening portion.

10. The scanning tip for the photoacoustic-ultrasonic mini-probe of claim 8, wherein the scanning tip casing has an epoxy injection hole adjacent to another end.

11. The scanning tip for the photoacoustic-ultrasonic mini-probe of claim 10, further comprising an epoxy configured to fill an inside of the scanning tip casing through the epoxy injection hole.
